# EUROPÄISCHE PATENTSCHRIFT

(11) **EP 0 504 872 B1**
(45) Veröffentlichungstag und Bekanntmachung des Hinweises auf die Patenterteilung: **08.11.1995**
(21) Anmeldenummer: 92104777.5
(22) Anmeldetag: 19.03.1992
(51) Int. Cl.: C09B 5/62, C09B 67/22, C09B 67/20, C08K 5/42, C08K 5/19

(54) **Innere Salze von Perylenverbindungen, Verfahren zu deren Herstellung und ihre Verwendung**
Inner salts of perylene compounds, process for their preparation and their use
Sels internes de pérylènes, méthode pour leur préparation et leur utilisation

(30) Priorität: 21.03.1991 DE 4109244
(43) Veröffentlichungstag der Anmeldung: 23.09.1992
(73) Patentinhaber: HOECHST AKTIENGESELLSCHAFT, 65926 Frankfurt am Main (DE)
(72) Erfinder: Dietz, Erwin, Dr., W-6233 Kelkheim (Taunus) (DE); Urban, Manfred, W-6200 Wiesbaden (DE)

(56) Entgegenhaltungen:
- EP-A- 0 039 482
- EP-A- 0 302 973
- WO-A-91/02032
- WO-A-91/02034

## Beschreibung

Die vorliegende Erfindung betrifft neue, wertvolle Perylenverbindungen, die als innere Salze vorliegen und der allgemeinen Formel I 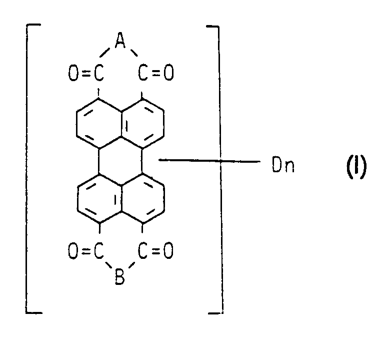
entsprechen, in welcher
- A: einen bivalenten Rest des Typs 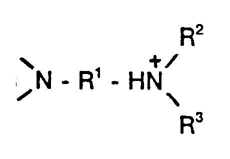 von kationischer Natur und
- B: einen bivalenten Rest des Typs 〉N-R⁴-SO₃⁻ von anionischer Natur bedeutet,
- n: einen Wert von 0 bis 8, vorzugsweise von 1 bis 6 hat, und wenn n > 0 ist,
- D: ein Chlor- oder Bromatom und sofern n > 1 ist ggf. eine Kombination davon darstellt,
wobei in den obigen Resten A und B dann
- R¹: für eine Alkylengruppe mit 1 - 12 C-Atomen, vorzugsweise 2 - 6 C-Atomen, eine Aralkylengruppe oder eine Arylengruppe, vorzugsweise Phenylen steht,
- R² und R³: einzeln für sich genommen sowie unabhängig voneinander jeweils ein Wasserstoffatom, einen substituierten oder unsubstituierten Alkylrest mit 1 - 20 C-Atomen, vorzugsweise 1 - 6 C-Atomen, oder einen substituierten oder unsubstituierten Alkenylrest mit 2 - 20 C-Atomen bedeuten, aber nicht beide gleichzeitig Wasserstoff sind, oder
- R² und R³: gemeinsam sowie zusammen mit dem angrenzenden N-Atom ein heterocyclisches System bilden, das ggf. noch weitere ringangehörige Heteroatome wie ein O-, S- und/oder N-Atom enthält und an das ggf. zusätzliche Ringe ankondensiert sind, und
- R⁴: eine geradkettige oder verzweigte Alkylengruppe mit 1 - 12 C-Atomen, vorzugsweise 1 - 6 C-Atomen darstellt.

Besonderes Interesse haben erfindungsgemäß die Verbindungen der allgemeinen Formel I gefunden, in denen die Bedeutungen für die Symbole A und B sowie D und n wie vorstehend angegeben charakterisiert sind, wobei aber innerhalb des strukturell ausgewiesenen Aufbauprinzips für die Reste A und B dann
- R¹: für die Ethylen- oder Propylengruppe steht,
- R² und R³: einzeln für sich genommen sowie unabhängig voneinander jeweils den Ethyl-, Propyl oder Butylrest bezeichnen, der ggf. noch nichtionische Substituenten enthält, vorzugsweise sind diese beiden Reste R² und R³ übereinstimmend, oder
- R² und R³: gemeinsam sowie zusammen mit dem angrenzenden N-Atom einen heterocyclischen Fünf- oder Sechsring von aliphatischer Natur mit jeweils 1 bis 3 ringangehörigen, gleichen oder unterschiedlichen Heteroatomen (in der Hauptsache Stickstoff) bilden, wie z.B. vom Pyrrolidin-, Piperazin-, und besonders Piperidin- oder Morpholin-Typ, oder
- R² und R³: auf analoge Weise einen entsprechenden Heteroring von aromatischer Natur bilden, wie z.B. vom Pyrrol-, Imidazolin- und besonders Imidazol-Typ, und ein derartiges Ringsystem beidemal nichtionische Substituenten enthält sowie im Falle der Heteroaromaten auch einen benzokondensierten Ring aufweisen kann, wie z.B. vom Indol-, Indolin- oder Benzimidazol-Typ, und
- R⁴: die Ethylen- oder Propylengruppe darstellt.

Ganz besondere Aufmerksamkeit im Zuge der Erfindung genießen wegen ihrer unmittelbaren praktischen Bedeutung diejenigen inneren Salze von Perylenverbindungen der allgemeinen Formel I, worin der Index n den Wert Null hat, also die halogenfreien Produkte der beanspruchten Kategorie.

Gegenstand der in Rede stehenden Erfindung ist auch ein Verfahren zur Herstellung der zuvor beschriebenen inneren Salze von Perylenverbindungen der allgemeinen Formel I. Diese neuen chemischen Produkte können nach zwei grundlegenden Verfahrensvarianten erhalten werden:
1) Durch Kondensation von Perylen-3,4,9,10-tetracarbonsäure-monoanhydrid-monoimiden, die am Imidstickstoff durch ein Strukturelement vom Typ 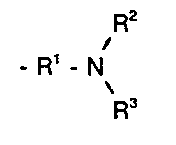 substituiert sind, in dem R¹, R² und R³ die oben angegebene Bedeutung besitzen, mit sulfonsäuregruppenhaltigen, primären aliphatischen Aminen der Formel II

   H₂N-R⁴-SO₃H (II),

   in der R⁴ wie vorstehend erläutert definiert ist, beispielsweise mit Aminoalkansulfonsäuren wie insbesondere Taurin (2-Aminoethansulfonsäure) bzw. deren Salzen, wobei man die Umsetzung der Ausgangsstoffe in wäßriger Lösung, unter alkalischen pH-Bedingungen, bei Temperaturen im Bereich zwischen 50° und 200°C, bevorzugt bei 100 - 150°C durchführt. Zweckmäßig werden hierbei die sulfonsäuregruppenhaltigen Amine der Formel II im Überschuß verwendet.
2) Durch Kondensation von Perylen-3,4,9,10-tetracarbonsäure-monoanhydrid-monoimiden, die am Imidstickstoff durch ein Strukturelement vom Typ -R⁴-SO₃H substituiert sind, in dem R⁴ wie vorstehend erläutert definiert ist, mit eine primäre Aminogruppe aufweisenden Alkylen-, Aralkylen- oder Arylendiaminen der Formel III 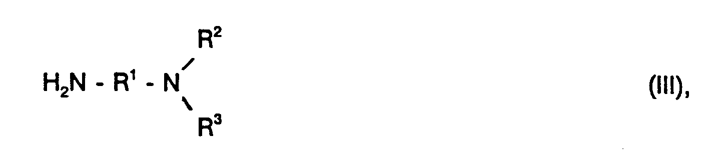 in der R¹, R² und R³ die oben angegebene Bedeutung besitzen, beispielsweise N-Alkylamino- bzw. N,N-Dialkylamino-alkylamine, wobei man die Umsetzung der Ausgangsstoffe in wäßriger Lösung, unter alkalischen Bedingungen, bei Temperaturen im Bereich zwischen 50°C und 200°C, bevorzugt bei 80 - 120°C durchführt. Die Diamine der Formel III werden zweckmäßigerweise im Überschuß eingesetzt.

Die Isolierung der nach den Verfahrensvarianten 1) und 2) gebildeten Verfahrensprodukte aus dem Reaktionsgemisch erfolgt durch Filtration ihrer Salze, welche hernach durch Behandlung mit starken Mineralsäuren wie Schwefelsäure in die inneren Salze gemäß Formel I umgewandelt werden.

Anstelle von in wäßrigem Medium kann die Kondensation der beiden Reaktionspartner nach der Variante 2) auch in hochsiedenden, inerten organischen Lösungsmitteln, z.B. aromatischen N-Heterocyclen, wie Pyridin, Picolin, Chinolin oder Imidazol, ferner in aromatischen Kohlenwasserstoffen wie Benzol oder Naphthalin, oder durch Alkyl, Hydroxy, Alkoxy oder Halogen substituiertes Benzol, besonders Toluol, Phenol, Anisol, Chlorbenzol, o-Dichlorbenzol oder 1,2,4-Trichlorbenzol, oder durch Alkyl oder Halogen substituiertes Naphthalin, besonders das Isomerengemisch Diisopropylnaphthalin oder α-Chlornaphthalin, ggf. unter Zusatz von bekannten Katalysatoren (Reaktionsbeschleunigern) wie Schwefelsäure, Phosphorsäure oder Zinksalzen, bei Temperaturen im Bereich zwischen 150° und 230°C, bevorzugt bei 180 - 210°C vorgenommen werden. Entsprechend dieser abgewandelten Synthesemöglichkeit filtriert man die als Salz entstandenen Perylenverbindungen in üblicher Weise aus dem Umsetzungsgemisch ab, ggf. nach vorherigem Verdünnen desselben mit inerten organischen Lösungsmitteln, z.B. aliphatischen Alkoholen oder aliphatischen Ketonen, und überführt sie anschließend durch Behandlung mit Mineralsäuren in die inneren Salze.

Halogenierte innere Salze von Perylenverbindungen der Formel I mit n > 0 sind erhältlich durch Kondensation von entsprechenden Halogenierungsprodukten (n > 0) der oben als Ausgangsverbindungen erwähnten Perylen-3,4,9,10-tetracarbonsäure-monoanhydrid-monoimiden eines jeden Typs mit sulfonsäuregruppenhaltigen Aminen der Formel II bzw. den Diamin-Abkömmlingen der Formel III; oder sie können durch nachträgliche Halogenierung bereits fertiggestellter, aber halogenfreier (n = 0) Verfahrensprodukte der Formel I bei der Einwirkung von elementarem Chlor oder Brom in hochkonzentrierter Schwefelsäure gewonnen werden.

Die Eigenschaften der beanspruchten, am gleichen Molekül sowohl anionische als auch kationische Strukturelemente aufweisenden inneren Salze von Perylenverbindungen der Formel I lassen sich durch spezielle Auswahl der vorhandenen Substituenten R¹, R², R³ und/oder R⁴ an den beiden Imidstickstoff-Funktionen bzw. der Anzahl n von Halogenatomen D am polycyclischen System in weiten Grenzen steuern. Das für den jeweiligen Anwendungszweck geforderte Eigenschaftsprofil dieser neuen Verbindungen muß durch orientierende Versuche ausgerichtet und gezielt optimiert werden.

Aus der EP-A-0 302 973 sind symmetrische Perylenverbindungen bekannt, die endständige basische Gruppen enthalten und als Pigmentdispergatoren verwendet werden. Ein Einsatz als Farbmittel ist nicht beschrieben.

In EP-A-0 039 482 sind Perylen-3,4,9,10-tetracarbonsäurernonoanhydrid-monoimide beschrieben, die insbesondere als Ausgangsstoffe für die Herstellung von Farbmitteln eingesetzt werden.

Die erfindungsgemäßen inneren Salze von Perylenverbindungen der Formel I haben sich als wertvolle Farbmittel erwiesen. Je nach der Natur und der daraus resultierenden Wirksamkeit der Substituenten an den beiden Imidstickstoffatomen kann man sie für diese Aufgabe unmittelbar als Pigment oder aber auch als Pigmentdispergator in Betracht ziehen.

Die für den Einsatz als Pigmente direkt brauchbaren inneren Salze von solchen Perylenverbindungen der Formel I können in einer transparenten oder einer deckenden Pigmentform bereitgestellt werden. Bei der Synthese anfallende Kondensationsprodukte dieses Typs sind an sich transparent, lassen sich aber durch die Wahl der Finishbedingungen und des Finishmediums in eine deckendere Pigmentform überführen, indem man das feuchte Rohpigment in Wasser oder organischen Lösungsmitteln unter Druck erwärmt.

Im Falle einer Verwendung der nach dieser Erfindung erhaltenen Verbindungen der Formel I als Dispergatoren für die Herstellung von Pigmentzubereitungen werden diese den Basispigmenten zur Modifizierung der Oberflächenstruktur derselben unter gleichmäßiger Beschichtung beigemischt. Vorzugsweise geschieht dies zusammen mit bekannten Perylenpigmenten, z.B. Pigment Red 224, Pigment Brown 26, Pigment Red 179, Pigment Red 189, Pigment Red 149, Pigment Red 190, Pigment Red 123 oder Pigment Red 178. Es lassen sich auf diese Weise aber auch Präparationen mit Basispigmenten von unterschiedlicher chemischer Herkunft wie Azo- und Chinacridonpigmenten realisieren. Die aus einem derartigen Anlaß den Basispigmenten zuzusetzenden Mengen an Pigmentdispergatoren sind - soweit davon die angestrebte Pigmentqualität nicht negativ beeinflußt wird - in Richtung auf einen bestimmten Maximalwert nicht beschränkt, doch kommt im allgemeinen ein Gehalt von 0,1 bis 20 Gew.-%, insbesondere von 1 bis 10 Gew.-% an Dispergator, berechnet auf das jeweilige Pigmentgewicht, in Frage.

Die unter Beteiligung der inneren Salze von Perylenverbindungen der Formel I erzeugten Pigmentzubereitungen können neben Pigment und Pigmentdispergatoren noch weitere Bestandteile, wie beispielsweise Tenside, Harze oder Antistaubmittel enthalten.

Die Herstellung von Pigmentzubereitungen kann im vorliegenden Fall auf verschiedene Weise erfolgen. So können die Pigmentdispergatoren schon im Stadium der Pigmentsynthese, im Rahmen eines Feinverteilungsprozesses oder einer anschließenden Lösungsmittelfinish-Behandlung eingetragen werden. Der Zusatz der Pigmentdispergatoren läßt sich beispielsweise im Verlauf einer Trockenmahlung eines Rohpigments mit oder ohne zusätzliche Mahlhilfsmittel auf einer Roll- oder Schwingmühle oder im Zuge einer Naßmahlung eines Rohpigments in wäßrigem, wäßrig-organischem oder organischem Mahlmedium, beispielsweise auf einer Perlmühle, durchführen. Gleichfalls bewährt hat sich die Zugabe der Pigmentdispergatoren vor oder während eines Druckfinishs für das zugrundeliegende Pigment in wäßrigem, wäßrig-saurem bzw. wäßrig-organischem Medium. Die Pigmentdispergatoren können auch dem wasserfeuchten Pigmentpreßkuchen vor der Trocknung zugesetzt und eingearbeitet werden. Es ist schließlich möglich, Trockenmischungen von gemahlenen Pigmentdispergatoren mit dem Pigmentpulver vorzunehmen.

Die als Pigment direkt einsetzbaren erfindungsgemäßen inneren Salze von Perylenverbindungen der Formel I sowie die damit in der Funktion als Dispergator erhältlichen Pigmentzubereitungen sind in vielen Anwendungsmedien leicht und bis zu hohen Feinheiten dispergierbar. Solche Dispersionen besitzen eine hohe Flockungsstabilität und zeigen hervorragende rheologische Eigenschaften selbst bei hoher Pigmentierung. Mit ihnen lassen sich Lackierungen und Drucke von hoher Farbstärke, hohem Glanz und hoher Transparenz mit ausgezeichneten Echtheitseigenschaften erzielen.

Die als Pigmente und Pigmentzubereitungen verwendbaren erfindungsgemäßen Verbindungen der Formel I lassen sich besonders zum Pigmentieren (Einfärben) von hochmolekularen organischen Materialien natürlicher oder synthetischer Herkunft einsetzen:
Hochmolekulare organische Materialien, die mit derartigen Pigmenten oder Pigmentzubereitungen coloriert werden können, sind beispielsweise Celluloseether und -ester, wie Ethylcellulose, Nitrocellulose, Celluloseacetat oder Cellulosebutyrat, natürliche Harze oder Kunstharze, wie Polymerisationsharze oder Kondensationsharze, z.B. Aminoplaste, insbesondere Harnstoff- und Melamin-Formaldehydharze, Alkydharze, Acrylharze, Phenoplaste, Polycarbonate, Polyolefine, wie Polystyrol, Polyvinylchlorid, Polyethylen, Polypropylen, Polyacrylnitril, Polyacrylsäureester, Polyamide, Polyurethane oder Polyester, Gummi, Casein, Silikon und Silikonharze, einzeln oder in Mischungen.
Dabei spielt es keine Rolle, ob die erwähnten hochmolekularen organischen Verbindungen als plastische Massen, Schmelzen oder in Form von Spinnlösungen, Lacken,Anstrichstoffen oder Druckfarben vorliegen. Je nach Verwendungszweck erweist es sich als vorteilhaft, die Pigmente oder Pigmentzubereitungen als Toner oder in Form von Präparationen oder Dispersionen zu benutzen. Bezogen auf das zu pigmentierende hochmolekulare organische Material setzt man solche Pigmente oder Pigmentzubereitungen in einer Menge von vorzugsweise 1 bis 10 Gew.-% ein.

Besonders bevorzugte Lacksysteme sind in dieser Hinsicht Einbrennlacke aus der Klasse der Alkyd-/Melaminharz- oder Acryl-/Melaminharz-Lacke sowie Zweikomponentenlacke auf Basis von mit Polyisocyanat vernetzbaren Acrylharzen. Von der Vielzahl der pigmentierbaren Dtuckfarben sind Druckfarben auf Basis Nitrocellulose besonders zu erwähnen.
Die Pigmente oder Pigmentzubereitungen auf erfindungsgemäßer Grundlage sind als polymerunlösliche Farbmittel auch hervorragend geeignet zum Einfärben von verformbaren Kunststoffen, insbesondere für Polyvinylchlorid, Polyethylen und Polypropylen. Es werden Färbungen von hoher Farbstärke und sehr guter Dispergierbarkeit erhalten.

Zur Prüfung der Eigenschaften von derartigen Pigmenten oder Pigmentzubereitungen auf dem Lacksektor wurden aus der Vielzahl der bekannten Systeme ein Alkyd-/Melaminharz-Lack (AM6) auf Basis eines mittelöligen, nichttrocknenden Alkydharzes aus synthetischen Fettsäuren und Phthalsäureanhydrid und eines mit Butanol veretherten Melaminharzes und Anteilen eines nichttrocknenden Alkydharzes auf Basis von Ricinensäure (kurzölig) sowie ein Acrylharz-Einbrennlack auf Basis einer nicht-wäßrigen Dispersion (TSA) ausgewählt. In den nachfolgenden Beispielen wird darauf unter der Bezeichnung AM6 bzw. TSA verwiesen.

Die Rheologie des Mahlguts nach der Dispergierung (Millbase-Rheologie) wird anhand der folgenden fünfstufigen Skala bewertet:

| | |
|---|---|
| 5 dünnflüssig | 2 leicht gestockt |
| 4 flüssig | 1 gestockt |
| 3 dickflüssig | |

Nach dem Verdünnen des Mahlgutes auf die Pigmentendkonzentration wurde die Viskosität mit dem Viskospatel nach Rossmann, Typ 301, der Firma Erichsen beurteilt.
Glanzmessungen erfolgten an Folienaufgüssen unter einem Winkel von 20° nach DIN 67530 (ASTMD 523) mit dem "multigloss"-Glanzmeßgerät der Firma Byk-Mallinckrodt.

In den nachstehenden Beispielen beziehen sich Teile jeweils auf Gewichtsteile und Prozente jeweils auf Gewichtsprozente der so beschriebenen Substanzen. Die zur Identifizierung benutzten Gattungsbezeichnungen für erfindungsgemäß eingesetzte Pigmente sowie darauf gerichtete C.I.-Nummern sind dem COLOUR INDEX, 3. Auflage 1971, Band 4, sowie Ergänzungen 1975, 1982 und 1987 entnommen. Das in den Beispielen als Ausgangsstoff verwendete Perylen-3,4,9,10-tetracarbonsäure-monoanhydrid-monosulfoethylimid bzw. -propylimid wurde nach der DE-OS 39 26 563 und die Perylen-3,4,9,10-tetracarbonsäure-monoanhydrid-mono-N-alkylaminoalkylimide wurden nach der EP-PS 0 039 482 hergestellt.

### Beispiel 1

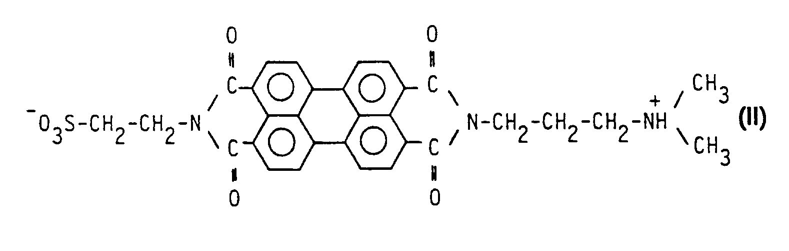

In einem Rührgefäß werden 9,98 g Perylen-3,4,9,10-tetracarbonsäure-monoanhydrid-mono-β-sulfoethylimid (99,0 %ig) in 300 ml Wasser suspendiert und zu dieser Suspension werden 8,16 g 3-Dimethylaminopropylamin gegeben, worauf man den Ansatz 5 Stunden auf 100°C erhitzt. Nach Abkühlen auf 25°C werden noch 40 g Kaliumchlorid eingebracht und das Gemisch wird weitere 2 Stunden bei 25°C nachgerührt. Das hierbei angefallene Produkt wird danach abgesaugt, mit 10 %iger Kaliumchloridlösung neutral gewaschen und bei 80°C getrocknet.

Der erhaltene Rückstand wird nun bei 25°C zunächst in 100 g 95 %ige Schwefelsäure eingetragen und darin gelöst. Sodann werden in diese Lösung weiterhin 155 g 48 %ige Schwefelsäure zugetropft, wobei die Temperatur bis 80°C ansteigen darf. Anschließend läßt man auf 25°C abkühlen; das abgeschiedene Salz wird über eine Glasfritte abgesaugt, mit 50 %iger Schwefelsäure nachgewaschen, mit Wasser neutral gewaschen und bei 80°C getrocknet.
Ausbeute: 11,87 g einer Verbindung mit der oben genannten Formel II, die 6,9 % Kristallwasser enthält, entsprechend 11,05 g 100 %ig (= 95,7 % d. Th.).
Analyse: C₃₁H₂₅N₃SO₇
Unter Berücksichtigung von 6,9 % H₂O

| | | | | |
|---|---|---|---|---|
| Ber.: | C 63,8 %, | H 4,3 %, | N 7,2 %, | S 5,5 % |
| Gef.: | C 63,4 %, | H 4,0 %, | N 6,9 %, | S 5,6 % |

¹H-NMR-Spektrum in D₂SO₄
Shift-Lage (¹H) 2,75; 3,1; 2,14; 4,16; 3,75; 4,79; 6,2

### Beispiel 1a

18,0 g Pigment Red 179 (C.I. Nr. 71130), hergestellt analog der EP-PS 0 088 392 unter Zusatz von 15 % Harzseife, werden mit 2 g Pigmentdispergator der obigen Formel II mechanisch gemischt.
Man erhält eine Pigmentzubereitung, die bei der Ausprüfung im TSA-Lack transparente und farbstarke Lackierungen mit hohem Glanz liefert. Die Millbase-Rheologie der Lackfarbe wird mit Note 3 - 4 eingestuft.

Wird das vorstehende Beispiel 1a wiederholt, aber ohne Zugabe des Pigmentdispergators, so ergibt die Rheologie der mit dem daraus resultierenden Produkt erzeugten Lackfarbe bloß die Note 1.

### Beispiel 2

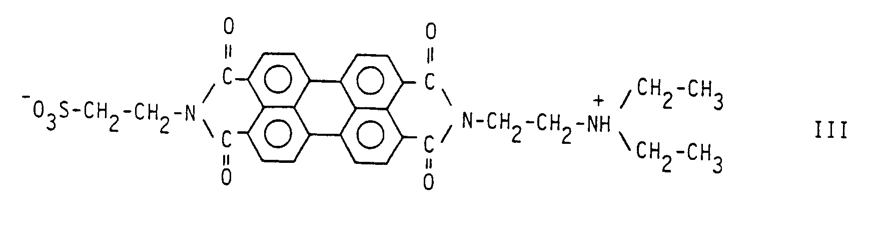

In einem Autoklaven werden 750 ml Wasser vorgelegt und mit 26,3 g Taurin sowie 13,9 g Kaliumhydroxid (85 %ig) beschickt. Danach trägt man noch 25,8 g Perylen-3,4,9,10-tetracarbonsäure-monoanhydrid-mono-β-diethylaminoethylimid ein, das Gemisch wird auf 150°C geheizt und 3 Stunden bei dieser Temperatur nachgerührt. Nach Abkühlen auf 25°C wird das ausgefallene Produkt abgesaugt, mit Wasser neutral gewaschen und bei 80°C getrocknet.

Der erhaltene Rückstand wird nun bei 25°C zunächst in 300 g 100 %ige Schwefelsäure eingebracht und darin gelöst. Sodann werden dieser Lösung außerdem 700 g 15 %ige Schwefelsäure tropfenweise zugefügt, wobei die Temperatur bis 80°C ansteigen darf. Anschließend läßt man auf 25°C abkühlen; das abgeschiedene Salz wird über eine Glasfritte abgesaugt, mit Wasser neutral gewaschen und bei 80°C getrocknet.
Ausbeute: 27,6 g einer Verbindung der oben genannten Formel III, die 3,0 % Kristallwasser enthält, entsprechend 26,8 g 100 %ig (= 85,4 % d. Th.)
Analyse C₃₂H₂₇N₃SO₇
Unter Berücksichtigung von 3,0 % H₂O

| | | | | |
|---|---|---|---|---|
| Ber.: | C 64,3 %, | H 4,5 %, | N 7,0 %, | S 5,4 % |
| Gef.: | C 64,0 %, | H 4,8 %, | N 6,4 %, | S 5,6 % |

¹H-NMR-Spektrum in D₂SO₄
Shift-Lage (¹H) 1,2; 3,2; 3,4; 4,6; 3,75; 4,79; 6,18

### Beispiel 2a

In einem Autoklaven werden 150 ml Wasser vorgelegt, mit 15 g Perylenverbindung der obigen Formel III vermischt, danach auf 150°C geheizt und 5 Stunden bei dieser Temperatur nachgerührt. Nach Abkühlen auf 25°C wird das gefinishte Erzeugnis abgesaugt, mit Wasser gewaschen und bei 80°C getrocknet.
Man erhält 13,2 g eines Pigments, das im AM6-Lack deckende Lackierungen von reiner, roter Nuance sowie mit hohem Glanz liefert. Die Millbase-Rheologie der Lackfarbe wird mit Note 5 bewertet. Die Überlackierechtheit ist einwandfrei. Die Viskosität des 5 %igen Lacks beträgt 5,9''.

### Beispiel 3

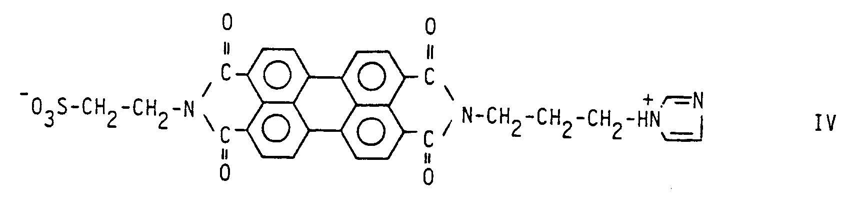

In einem Autoklaven werden 1300 ml Wasser vorgelegt und mit 47,8 g Taurin sowie 25,2 g Kaliumhydroxid (85 %ig) versetzt. Danach werden noch 47,6 g Perylen-3,4,9,10-tetracarbonsäure-monoanhydrid-mono-γ-imidazolylpropylimid hinzugefügt, worauf man das Gemisch auf 150°C heizt und 3 Stunden bei dieser Temperatur nachrühren läßt. Nach Abkühlen auf 25°C wird das ausgefallene Produkt abgesaugt, mit Wasser neutral gewaschen und bei 80°C getrocknet.

Der erhaltene Rückstand wird nun bei 25°C zunächst in 700 g 100 %ige Schwefelsäure eingetragen und darin gelöst. In diese Lösung werden sodann 1600 g 15 %ige Schwefelsäure getropft, wobei die Temperatur bis 80°C ansteigen darf. Anschließend läßt man auf 25°C abkühlen; das abgeschiedene Salz wird über eine Glasfritte abgesaugt, mit Wasser neutral gewaschen und bei 80°C getrocknet.
Ausbeute: 53,5 g einer Verbindung der oben genannten Formel IV, die 2,2 % Kristallwasser enthält, entsprechend 52,3 g 100 %ig (= 90,5 % d. Th.).
Analyse C₃₂H₂₂N₄SO₇
Unter Berücksichtigung von 2,2 % H₂O

| | | | | |
|---|---|---|---|---|
| Ber.: | C 63,4 %, | H 3,6 %, | N 9,2 %, | S 5,3 % |
| Gef.: | C 62,8 %, | H 3,7 %, | N 9,2 %, | S 5,5 % |

### Beispiel 4

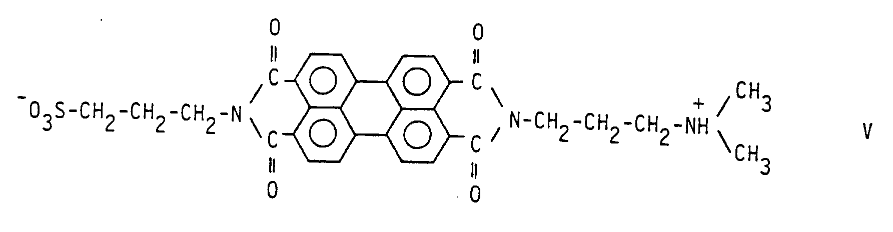

In einem Rührgefäß werden 5,40 g Perylen-3,4,9,10-tetracarbonsäure-monoanhydrid-mono-γ-sulfopropylimid (94,0 %ig) in 100 ml Wasser suspendiert und zu dieser Suspension werden 4,10 g 3-Dimethylaminopropylamin gegeben, worauf man den Ansatz 5 Stunden auf 100°C erhitzt. Nach Abkühlen auf 25°C werden noch 20 g Kaliumchlorid zugefügt und das Gemisch wird 1 Stunde bei 25°C nachgerührt. Das hierbei ausgefallene Produkt wird danach abgesaugt, mit 10 %iger Kaliumchloridlösung neutral gewaschen und bei 80°C getrocknet.

Der erhaltene Rückstand wird nun bei 25°C zunächst in 100 g 100 %ige Schwefelsäure eingetragen und darin gelöst. Sodann werden in diese Lösung weitere 260 g 12,5 %ige Schwefelsäure getropft, wobei die Temperatur bis 80°C ansteigen darf. Anschließend läßt man auf 25°C abkühlen; das abgeschiedene Salz wird über eine Glasfritte abgesaugt, mit 10 %iger Schwefelsäure nachgewaschen, mit Wasser neutral gewaschen und bei 80°C getrocknet.
Ausbeute: 5,14 g einer Verbindung der oben genannten Formel V, die 3,7 % Kristallwasser enthält, entsprechend 5,20 g 100 %ig (= 88,0 % d. Th.)
Analyse C₃₂H₂₇N₃SO₇
Unter Berücksichtigung von 3,7 % H₂O

| | | | | |
|---|---|---|---|---|
| Ber.: | C 64,3 %, | H 4,5 %, | N 7,0 %, | S 5,4 % |
| Gef.: | C 63,9 %, | H 4,5 %, | N 6,9 %, | S 5,6 % |

### Beispiel 5

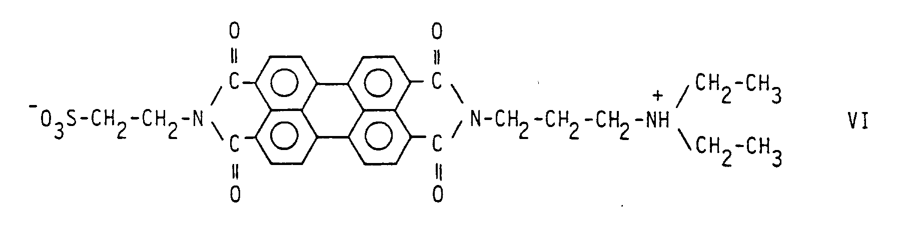

In einem Autoklaven werden 200 ml Wasser vorgelegt und danach mit 10,0 g Perylen-3,4,9,10-tetracarbonsäure-monoanhydrid-mono-β-sulfoethylimid (98,4 %ig) sowie 10,4 g 3-Diethylaminopropylamin versetzt. Anschließend wird das Gemisch auf 125°C geheizt und 5 Stunden bei dieser Temperatur nachgerührt. Sodann läßt man es auf 25°C abkühlen, worauf mittels 20,3 g Salzsäure (31 %ig) der pH-Wert desselben auf 1,2 eingestellt wird. Nach 1 Stunde weiterem Nachrühren bei 25°C wird das abgeschiedene Salz schließlich abgesaugt, mit Wasser neutral gewaschen und bei 80°C getrocknet.
Ausbeute: 11,6 g einer Verbindung der oben genannten Formel VI, die 3,7 % Kristallwasser enthält, entsprechend 11,2 g 100 %ig (= 93,3 % d. Th.)
Analyse C₃₃H₂₉N₃SO₇
Unter Berücksichtigung von 3,7 % H₂O

| | | | | |
|---|---|---|---|---|
| Ber.: | C 64,8 %, | H 4,8 %, | N 6,9 %, | S 5,2 % |
| Gef.: | C 64,0 %, | H 4,6 %, | N 6,8 %, | S 5,2 % |

### Beispiel 6

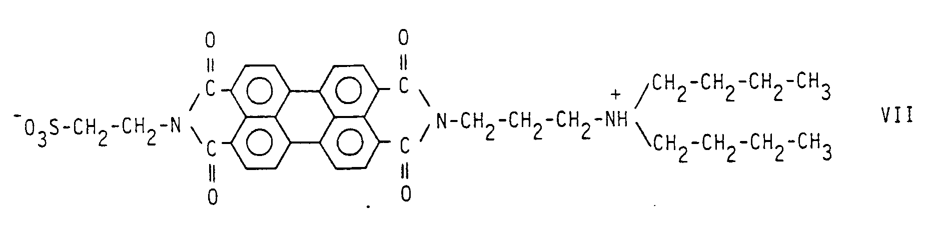

In einem Rührgefäß werden 50 ml Chinolin vorgelegt und nacheinander mit 4,99 g Perylen-3,4,9,10-tetracarbonsäure-monoanhydrid-mono-β-sulfoethylimid (99,2 %ig), 7,5 g 3-Dibutylaminopropylamin sowie 0,5 g Zinkacetat-dihydrat versetzt. Danach wird das Gemisch auf 190°C erhitzt und 8 Stunden bei dieser Temperatur nachgerührt. Man läßt es daraufhin 120°C abkühlen, das ausgefallene Produkt wird abgesaugt, mit Chinolin und Methanol gewaschen und bei 80°C getrocknet.

Der obige Rückstand wird nun bei 25°C zunächst in 45 g 100 %ige Schwefelsäure eingetragen und darin gelöst. In diese Lösung werden sodann 50 ml Wasser getropft, wobei die Temperatur bis 80°C ansteigen darf. Anschließend wird das hierbei abgeschiedene Salz über eine Glasfritte abgesaugt, mit 50 %iger Schwefelsäure nachgewaschen, mit Wasser neutral gewaschen und bei 80°C getrocknet.
Ausbeute: 3,94 g einer Verbindung der oben genannten Formel VII, die 2,1 % Kristallwasser enthält, entsprechend 3,86 g 100 %ig (= 58,3 % d. Th.)
Analyse C₃₇H₃₇N₃SO₇
Unter Berücksichtigung von 2,1 % H₂O

| | | | | |
|---|---|---|---|---|
| Ber.: | C 66,6 %, | H 5,6 %, | N 6,3 %, | S 4,8 % |
| Gef.: | C 67,3 %, | H 5,3 %, | N 6,3 %, | S 5,3 % |

### Beispiel 7

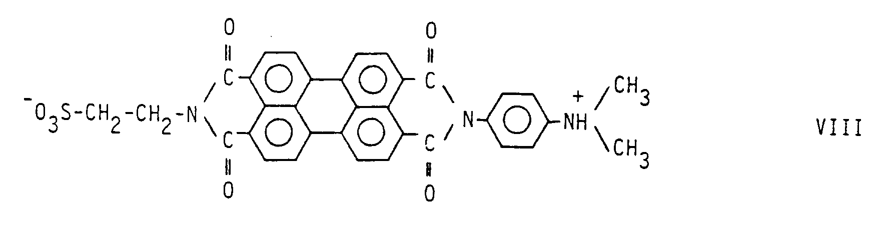

In einem Rührgefäß werden 50 ml Chinolin vorgelegt und nacheinander mit 4,99 g Perylen-3,4,9,10-tetracarbonsäure-monoanhydrid-mono-β-sulfoethylimid (99,2 %ig), 5,45 g N,N-Dimethyl-p-phenylendiamin sowie 0,5 g Zinkacetat-dihydrat versetzt. Danach wird das Gemisch auf 190°C erhitzt und 8 Stunden bei dieser Temperatur nachgerührt. Man läßt es daraufhin auf 25°C abkühlen, das ausgefallene Produkt wird abgesaugt, mit Chinolin, Methanol und Wasser gewaschen und bei 80°C getrocknet.

Der obige Rückstand wird nun bei 25°C zunächst in 70 g 100 %ige Schwefelsäure eingetragen und darin gelöst. In diese Lösung werden sodann 150 g 15 %ige Schwefelsäure getropft, wobei die Temperatur bis 80°C ansteigen darf. Anschließend wird das hierbei abgeschiedene Salz bei 25°C über eine Glasfritte abgesaugt, mit 10 %iger Schwefelsäure nachgewaschen, mit Wasser neutral gewaschen und bei 80°C getrocknet.
Ausbeute: 5,55 g einer Verbindung der oben genannten Formel VIII, die 2,1 % Kristallwasser enthält, entsprechend 5,43 g 100 %ig (= 88,7 % d. Th.)
Analyse C₃₄H₂₃N₃SO₇
Unter Berücksichtigung von 2,1 % H₂O

| | | | | |
|---|---|---|---|---|
| Ber.: | C 66,1 %, | H 3,7 %, | N 6,8 %, | S 5,2 % |
| Gef.: | C 65,0 %, | H 4,0 %, | N 6,5 %, | S 5,6 % |

### Beispiel 8

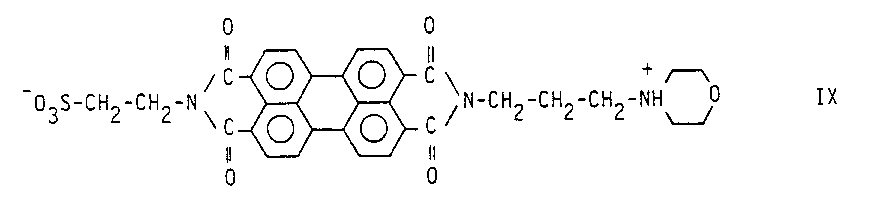

In einem Rührgefäß werden 50 ml Chinolin vorgelegt und nacheinander mit 4,99 g Perylen-3,4,9,10-tetracarbonsäure-monoanhydrid-mono-β-sulfoethylimid (99,2 %ig), 5,77 g N-(3-Aminopropyl)-morpholin sowie 0,5 g Zinkacetat-dihydrat versetzt. Danach wird das Gemisch auf 190°C erhitzt und 8 Stunden bei dieser Temperatur nachgerührt. Man läßt es daraufhin auf 25°C abgekühlen, das ausgefallene Produkt wird abgesaugt, mit Chinolin, Methanol und Wasser gewaschen und bei 80°C getrocknet.

Der obige Rückstand wird nun bei 25°C zunächst in 50 g 100 %ige Schwefelsäure eingetragen und darin gelöst. In diese Lösung werden sodann 50 ml Wasser getropft, wobei die Temperatur bis 80°C ansteigen darf. Anschließend wird das hierbei abgeschiedene Salz bei 25°C über eine Glasfritte abgesaugt, mit 50 %iger Schwefelsäure nachgewaschen, mit Wasser neutral gewaschen und bei 80°C getrocknet.
Ausbeute: 4,10 g einer Verbindung der oben genannten Formel IX, die 2,0 % Kristallwasser enthält, entsprechend 4,02 g 100 %ig (= 64,8 % d. Th.)
Analyse C₃₃H₂₇N₃SO₈
Unter Berücksichtigung von 2,0 % H₂O

| | | | | |
|---|---|---|---|---|
| Ber.: | C 63,4 %, | H 4,3 %, | N 6,7 %, | S 5,1 % |
| Gef.: | C 62,6 %, | H 4,5 %, | N 6,6 %, | S 5,8 % |

### Beispiel 9

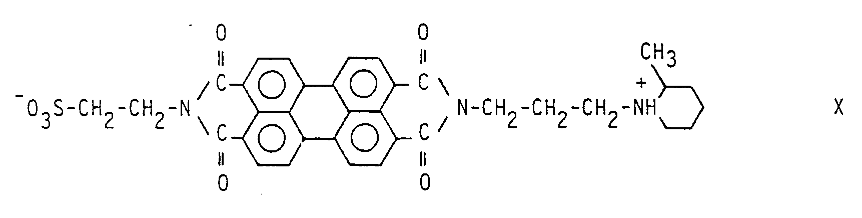

In einem Rührgefäß werden nacheinander 40 ml 1-(3-Aminopropyl)-2-methylpiperidin, 4,99 g Perylen-3,4,9,10-tetracarbonsäure-monoanhydrid-mono-β-sulfoethylimid sowie 0,5 g Zinkacetat-dihydrat eingetragen. Danach wird das Gemisch auf 190°C erhitzt und 8 Stunden bei dieser Temperatur nachgerührt. Man läßt es daraufhin auf 25°C abkühlen, das ausgefallene Produkt wird abgesaugt, mit Methanol und Wasser gewaschen und bei 80°C getrocknet.

Der obige Rückstand wird nun bei 25°C zunächst in 70 g 100 %ige Schwefelsäure eingetragen und darin gelöst. In diese Lösung werden sodann 150 g 15 %ige Schwefelsäure getropft, wobei die Temperatur bis 80°C ansteigen darf. Das hierbei abgeschiedene Salz wird bei 25°C über eine Glasfritte abgesaugt, mit 50 %iger Schwefelsäure nachgewaschen, mit Wasser neutral gewaschen und bei 80°C getrocknet.
Ausbeute: 5,19 g einer Verbindung der oben genannten Formel X, die 2,0 % Kristallwasser enthält, entsprechend 5,09 g 100 %ig (= 80,5 % d. Th.)
Analyse C₃₅H₃₁N₃SO₇
Unter Berücksichtigung von 2,0 % H₂O

| | | | | |
|---|---|---|---|---|
| Ber.: | C 65,9 %, | H 4,9 %, | N 6,6 %, | S 5,0 % |
| Gef.: | C 65,6 %, | H 4,8 %, | N 6,6 %, | S 5,2 % |

### Beispiel 10

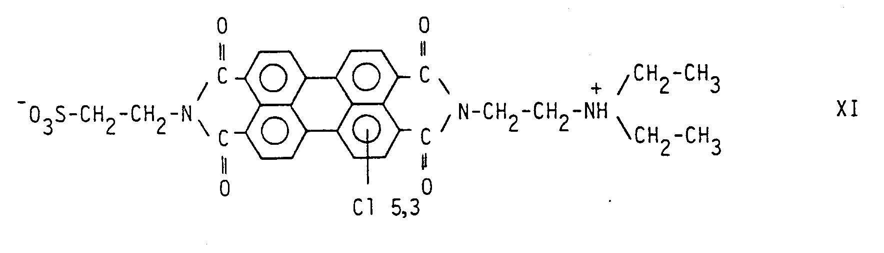

15 g Perylenverbindung der Formel III, hergestellt gemäß Beispiel 2, werden unter Rühren bei 25°C in 450 g 100 %ige Schwefelsäure eingetragen und darin gelöst. Nachdem man in diese Lösung 0,6 g Natriumjodid zugegeben hat, werden nun innerhalb von 6 Stunden 70 g Chlor bei 25°C eingeleitet, wobei man nach Ablauf von 3 Stunden nochmals 0,6 g Natriumjodid nachsetzt. Daraufhin werden dem Chlorierungsgemisch unter Rühren bei 25°C tropfenweise 435 ml Wasser zugefügt. Das hierbei ausgefallene Reaktionsprodukt wird über eine Glasfritte abgesaugt, durch Behandlung mit Wasser neutral gewaschen und bei 80°C getrocknet.
Ausbeute: 19,2 g einer Verbindung der oben genannten Formel XI, die 2,1 % Kristallwasser enthält, entsprechend 18,80 g 100 %ig (= 95,9 % d. Th.)
Analyse, bezogen auf Chlorgehalt laut Formel XI:
Unter Berücksichtigung von 2,1 % H₂O

| | | | | | |
|---|---|---|---|---|---|
| Ber.: | C 49,2 %, | H 2,8 %, | N 5,4 %, | S 4,1 %, | Cl 24,1 % |
| Gef.: | C 48,9 %, | H 2,8 %, | N 5,3 %, | S 3,9 %, | Cl 24,6 % |

Das Pigment ist orangefarben und liefert in TSA-Lacken transparente, farbstarke Lackierungen von reiner Nuance sowie mit hohem Glanz. Die Überlackierechtheit ist einwandfrei.

### Beispiel 11

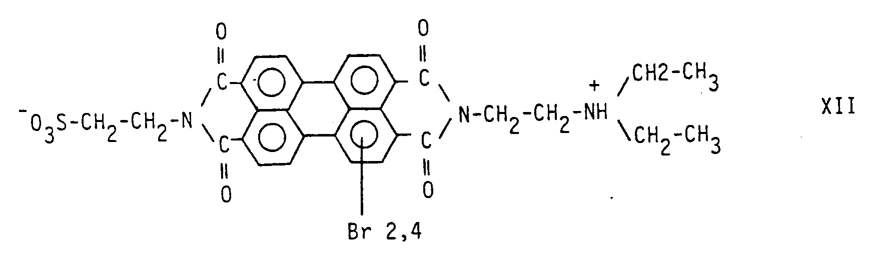

15 g Perylenverbindung der Formel III, hergestellt gemäß Beispiel 2, werden unter Rühren bei 25°C in 450 g 100 %ige Schwefelsäure eingetragen und darin gelöst. Dieser Lösung werden sodann 6 g Brom und 0,2 g Jod zugefügt. Anschließend wird das Bromierungsgemisch auf 125°C geheizt und noch 6 Stunden bei dieser Temperatur nachgerührt. Man läßt nun auf 25°C abkühlen und tropft bei dieser Temperatur 420 ml Wasser hinzu. Das hierbei ausgefallene Umsetzungsprodukt wird über eine Glasfritte abgesaugt, durch Behandlung mit Wasser neutral gewaschen und bei 80°C getrocknet.
Ausbeute: 19,3 g einer Verbindung der oben genannten Formel XII, die 1,4 % Kristallwasser enthält, entsprechend 19,0 g 100 %ig (= 96,2 % d. Th.)
Analyse, bezogen auf Bromgehalt laut Formel XII:
Unter Berücksichtigung von 1,4 % H₂O

| | | | | | |
|---|---|---|---|---|---|
| Ber.: | C 48,8 %, | H 3,1 %, | N 5,3 %, | S 4,1 %, | Br 24,4 % |
| Gef.: | C 48,8 %, | H 3,2 %, | N 5,0 %, | S 3,9 %, | Br 24,6 % |

Das Pigment ist rot und liefert in TSA-Lacken transparente, farbstarke Lackierungen von reiner Nuance sowie mit hohem Glanz. Die Überlackierechtheit ist einwandfrei.

## Patentansprüche

1. Innere Salze von Perylenverbindungen der allgemeinen Formel I 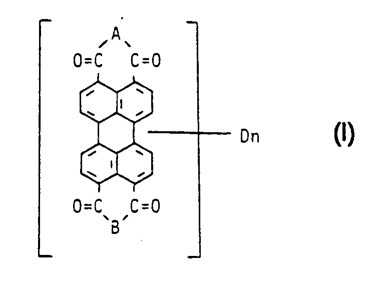 in welcher
A einen bivalenten Rest des Typs 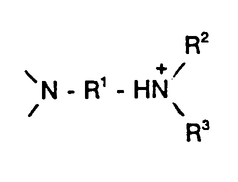 und
B einen bivalenten Rest des Typs 〉N-R⁴-SO₃⁻ bedeutet,
n einen Wert von 0 bis 8 hat, und wenn n > 0 ist,
D ein Chlor- oder Bromatom und sofern n > 1 ist ggf. eine Kombination davon darstellt;
wobei in den obigen Resten A und B dann
R¹ für eine Alkylengruppe mit 1 - 12 C-Atomen, eine Aralkylengruppe oder eine Arylengruppe steht,
R² und R³ einzeln für sich genommen sowie unabhängig voneinander jeweils ein Wasserstoffatom, einen substituierten oder unsubstituierten Alkylrest mit 1 - 20 C-Atomen oder einen substituierten oder unsubstituierten Alkenylrest mit 2 - 20 C-Atomen bedeuten, aber nicht beide gleichzeitig Wasserstoff sind, oder
R² und R³ gemeinsam sowie zusammen mit dem angrenzenden N-Atom ein heterocyclisches System bilden, das ggf. noch weitere ringangehörige Heteroatome enthält und an das ggf. zusätzliche Ringe ankondensiert sind, und
R⁴ eine geradkettige oder verzweigte Alkylengruppe mit 1 - 12 C-Atomen darstellt.

2. Innere Salze von Perylenverbindungen gemäß Anspruch 1, dadurch gekennzeichnet, daß in der dort angegebenen allgemeinen Formel I die Bedeutungen für die Symbole
A und B sowie D und n wie in Anspruch 1 charakterisiert sind,
wobei aber innerhalb des strukturell ausgewiesenen Aufbauprinzips für die Reste A und B dann
R¹ für eine C₂-C₆-Alkylengruppe, insbesondere die Ethylen- oder Propylengruppe steht,
R² und R³ einzeln für sich genommen sowie unabhängig voneinander jeweils einen C₁-C₆-Alkylrest, insbesondere den Ethyl-, Propyl- oder Butylrest bezeichnen, der ggf. noch nichtionische Substituenten enthält, oder
R² und R³ gemeinsam sowie zusammen mit dem angrenzenden N-Atom einen aliphatischen oder aromatischen, heterocyclischen Fünf- oder Sechsring mit jeweils 1 bis 3 ringangehörigen, gleichen oder unterschiedlichen Heteroatomen bilden und ein derartiges Ringsystem nichtionische Substituenten enthält sowie im Falle von aromatischer Natur auch einen benzokondensierten Ring aufweisen kann, und
R⁴ eine geradkettige oder verzweigte C₂-C₆-Alkylengruppe, insbesondere die Ethylen- oder Propylengruppe darstellt.

3. Innere Salze von Perylenverbindungen gemäß Anspruch 1 oder 2, dadurch gekennzeichnet, daß in der dort angegebenen allgemeinen Formel I die Bedeutungen für die Symbole
A und B wie in den Ansprüchen 1 oder 2 näher definiert sind und
n den Wert Null hat.

4. Innere Salze von Perylenverbindungen gemäß Anspruch 1 oder 2, dadurch gekennzeichnet, daß in der dort angegebenen allgemeinen Formel I die Bedeutungen für die Symbole
A und B wie in den Ansprüchen 1 oder 2 näher definiert sind,
n für einen Wert von 1 bis 6 steht und
D ein Chlor- und/oder Bromatom darstellt.

5. Verfahren zur Herstellung der gemäß einem oder mehreren der Ansprüche 1 bis 4 definierten inneren Salze von Perylenverbindungen der allgemeinen Formel I, dadurch gekennzeichnet, daß man am Imidstickstoff durch ein Strukturelement vom Typ 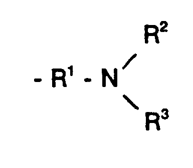 substituierte Perylen-3,4,9,10-tetracarbonsäure-monoanhydrid-monoimide, worin den Symbolen R¹, R² und R³ jeweils die entsprechend Formel I dafür aus den vorstehenden Ansprüchen ersichtliche Bedeutung zukommt, mit sulfonsäuregruppenhaltigen, primären aliphatischen Aminen der Formel II
H₂N-R⁴-SO₃H (II),
worin R⁴ mit der entsprechend Formel I für dieses Symbol aus den vorstehenden Ansprüchen ersichtlichen Bedeutung identisch ist, in wäßriger Lösung, unter alkalischen pH-Bedingungen, bei Temperaturen im Bereich zwischen 50° und 200°C, bevorzugt bei 100 - 150°C umsetzt, und daß man die angefallenen Kondensationsprodukte, zweckmäßig nach erfolgter Zwischenisolierung, durch Behandlung mit starken Mineralsäuren in die jeweiligen inneren Salze der Formel I überführt.

6. Verfahren zur Herstellung der gemäß einem oder mehreren der Ansprüche 1 bis 4 definierten inneren Salze von Perylenverbindungen der allgemeinen Formel I, dadurch gekennzeichnet, daß man am Imidstickstoff durch ein Strukturelement vom Typ -R⁴-SO₃H substituierte Perylen-3,4,9,10-tetracarbonsäure-monoanhydrid-monoimide, worin dem Symbol R⁴ die entsprechend Formel I dafür aus den vorstehenden Ansprüchen ersichtliche Bedeutung zukommt, mit eine primäre Aminogruppe aufweisenden Alkylen-, Aralkylen- oder Arylendiaminen der Formel III 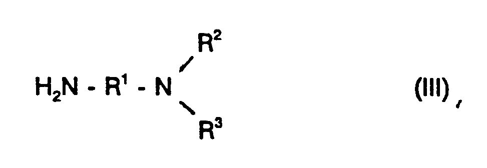 worin R¹, R² und R³ jeweils mit der entsprechend Formel I für diese Symbole aus den vorstehenden Ansprüchen ersichtlichen Bedeutung identisch sind, in wäßriger Lösung, unter alkalischen pH-Bedingungen, bei Temperaturen im Bereich zwischen 50° und 200°C, bevorzugt bei 80 - 120°C umsetzt, und daß man die angefallenen Kondensationsprodukte, zweckmäßig nach erfolgter Zwischenisolierung, durch Behandlung mit starken Mineralsäuren in die jeweiligen inneren Salze der Formel I überführt.

7. Verfahren zur Herstellung der gemäß einem oder mehreren der Ansprüche 1 bis 4 definierten inneren Salze von Perylenverbindungen der allgemeinen Formel I, dadurch gekennzeichnet, daß man am Imidstickstoff durch ein Strukturelement vom Typ - R⁴ - SO₃H substituierte Perylen-3,4,9,10-tetracarbonsäure-monoanhydrid-monoimide, worin dem Symbol R⁴ die entsprechend Formel I dafür aus den vorstehenden Ansprüchen ersichtliche Bedeutung zukommt, mit eine primäre Aminogruppe aufweisenden Alkylen-, Aralkylen- oder Arylendiaminen der Formel III 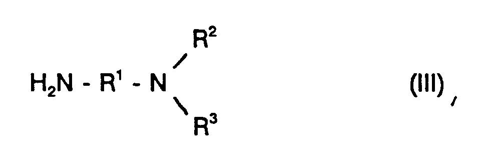 worin R¹, R² und R³ jeweils mit der entsprechend Formel I für diese Symbole aus den vorstehenden Ansprüchen ersichtlichen Bedeutung identisch sind, in hochsiedenden inerten organischen Lösemitteln bei Temperaturen im Bereich zwischen 150° und 230°C, bevorzugt bei 180 - 210°C, ggf. in Gegenwart von Katalysatoren umsetzt, und daß man die angefallenen Kondensationsprodukte, zweckmäßig nach erfolgter Zwischenisolierung, durch Behandlung mit starken Mineralsäuren in die jeweiligen inneren Salze der Formel I überführt.

8. Verwendung der gemäß einem oder mehreren der Ansprüche 1 bis 4 definierten inneren Salze von Perylenverbindungen der allgemeinen Formel I als Farbmittel zum Pigmentieren von hochmolekularen organischen Materialien natürlicher oder synthetischer Herkunft in Form plastischer Massen, Schmelzen, Spinnlösungen, Lacken, Anstrichfarben oder Druckfarben.

9. Verwendung der gemäß einem oder mehreren der Ansprüche 1 bis 4 definierten inneren Salze von Perylenverbindungen der allgemeinen Formel I als Pigmentdispergatoren zur Herstellung von Pigmentzubereitungen.

10. Verwendung von Pigmentzubereitungen gemäß Anspruch 9, deren Basispigment sich von der dem eingesetzten Dispergator zugrundeliegenden Perylen-Verbindungsklasse oder einem Derivat davon ableitet.

11. Verwendung von gemäß einem oder beiden der Ansprüche 9 und 10 definierten Pigmentzubereitungen als Farbmittel zum Pigmentieren von hochmolekularen organischen Materialien natürlicher oder synthetischer Herkunft in Form plastischer Massen, Schmelzen, Spinnlösungen, Lacken, Anstrichfarben oder Druckfarben.

## Claims

1. An inner salt of a perylene compound of the formula I 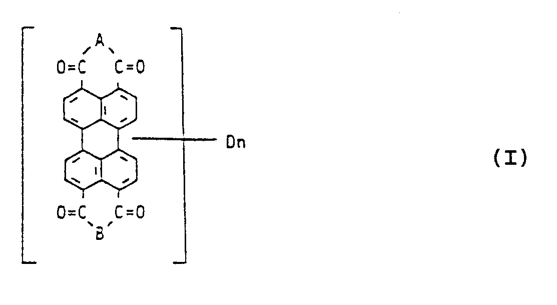 in which
A is a bivalent radical of the 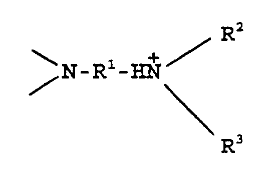 type, and
B is a bivalent radical of the 〉N-R⁴-SO₃⁻ type,
n has a value from 0 to 8, and, if n is > 0,
D is a chlorine or bromine atom and, if n is > 1, may be a combination thereof;
in which in the above radicals A and B
R¹ is an alkylene group having 1-12 carbon atoms, an aralkylene group or an arylene group,
R² and R³ individually and independently of one another are each a hydrogen atom, a substituted or unsubstituted alkyl radical having 1-20 carbon atoms, or a substituted or unsubstituted alkenyl radical having 2-20 carbon atoms but both are not simultaneously hydrogen, or
R² and R³ jointly and together with the adjacent nitrogen atom form a heterocyclic system which, if desired, contains further hetero atoms belonging to the ring and to which, if desired, additional rings are fused on, and
R⁴ is a straight-chain or branched alkylene group having 1-12 carbon atoms.

2. An inner salt of a perylene compound as claimed in claim 1, wherein in the formula I given there the meanings of
A and B and D and n are as defined in claim 1, in which however, within the structural principle for radicals A and B,
R¹ is a C₂-C₆-alkylene group, in particular an ethylene or propylene group,
R² and R³ individually and independently of one another are each a C₁-C₆-alkyl radical, in particular an ethyl, propyl or butyl radical,which, if desired, additionally contains nonionic substituents, or
R² and R³ jointly and together with the adjacent nitrogen atom form an aliphatic or aromatic heterocyclic five- or six-membered ring each having 1 to 3 identical or different hetero atoms belonging to the ring, and such a ring system contains nonionic substituents and if aromatic in nature can also contain a benzo-fused ring, and
R⁴ is a straight-chain or branched C₂-C₆-alkylene group, in particular an ethylene or propylene group.

3. An inner salt of a perylene compound as claimed in claim 1 or 2, wherein in the formula I given there the meanings of
A and B are as defined in more detail in claims 1 or 2 and
n has the value zero.

4. An inner salt of a perylene compound as claimed in claim 1 or 2, wherein in the formula I given there the meanings of
A and B are as defined in more detail in claims 1 or 2,
n is a value from 1 to 6 and
D is a chlorine and/or bromine atom.

5. A process for the preparation of an inner salt of a perylene compound of the formula I defined as claimed in one or more of claims 1 to 4, which comprises reacting perylene-3,4,9,10-tetracarboxylic monoanhydride monoimides which are substituted on the imide nitrogen by a structural element of the 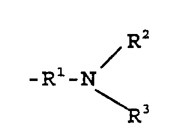 type and in which the symbols R¹, R² and R³ each have the meaning which can be seen therefor in accordance with formula I from the preceding claims, with sulfo-containing, primary aliphatic amines of the formula II
H₂N-R⁴-SO₃H (II),
in which R⁴ is identical to the meaning which can be seen in accordance with formula I for this symbol from the preceding claims, in aqueous solution, under alkaline pH conditions, at temperatures in the range between 50° and 200°C, preferably at 100-150°C, and converting the condensation products formed, advantageously after isolation of the intermediate, into the corresponding inner salts of the formula I by treatment with strong mineral acids.

6. A process for the preparation of an inner salt of a perylene compound of the formula I defined as claimed in one or more of claims 1 to 4, which comprises reacting perylene-3,4,9,10-tetracarboxylic monoanhydride monoimides which are substituted on the imide nitrogen by a structural element of the -R⁴-SO₃H type and in which the symbol R⁴ has the meaning which can be seen therefor in accordance with formula I from the preceding claims, with alkylene-, aralkylene- or arylenediamines having a primary amino group of the formula III 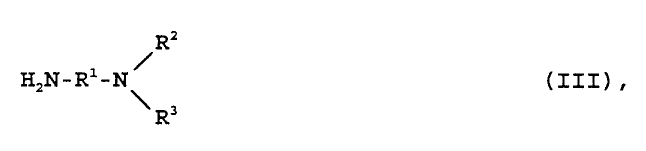 in which R¹, R² and R³ are each identical to the meaning which can be seen in accordance with formula I for these symbols from the preceding claims, in aqueous solution, under alkaline pH conditions, at temperatures in the range between 50° and 200°C, preferably at 80-120°C, and converting the condensation products formed, advantageously after isolation of the intermediate, into the corresponding inner salts of the formula I by treatment with strong mineral acids.

7. A process for the preparation of an inner salt of a perylene compound of the formula I defined as claimed in one or more of claims 1 to 4, which comprises reacting perylene-3,4,9,10-tetracarboxylic monoanhydride monoimides which are substituted on the imide nitrogen by a structural element of the -R⁴-SO₃H type and in which the symbol R⁴ has the meaning which can be seen therefor in accordance with formula I from the preceding claims, with alkylene-, aralkylene- or arylenediamines having a primary amino group of the formula III 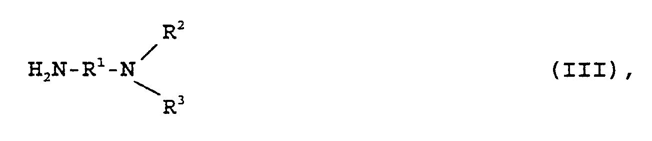 in which R¹, R² and R³ are each identical to the meaning which can be seen in accordance with formula I for these symbols from the preceding claims, in high-boiling inert organic solvents at temperatures in the range between 150° and 230°C, preferably at 180-210°C, if appropriate in the presence of catalysts and converting the condensation products formed, advantageously after isolation of the intermediate, into the corresponding inner salts of the formula I by treatment with strong mineral acids.

8. Use of an inner salt of a perylene compound of the formula I defined as claimed in one or more of claims 1 to 4 as colorant for pigmenting high-molecular-weight organic materials of natural or synthetic origin in the form of plastic compositions, melts, spinning solutions, varnishes, paints or printing inks.

9. Use of an inner salt of a perylene compound of the formula I defined as claimed in one or more of claims 1 to 4 as pigment dispersant for the preparation of a pigment preparation.

10. Use of a pigment preparation as claimed in claim 9, whose base pigment is derived from the perylene class of compounds on which the dispersant used is based or a derivative thereof.

11. Use of a pigment preparation defined as claimed in one or both of claims 9 and 10 as colorant for pigmenting high-molecular-weight organic materials of natural or synthetic origin in the form of plastic compositions, melts, spinning solutions, varnishes, paints or printing inks.

## Revendications

1. Sels internes de composés de pérylène de formule générale I : 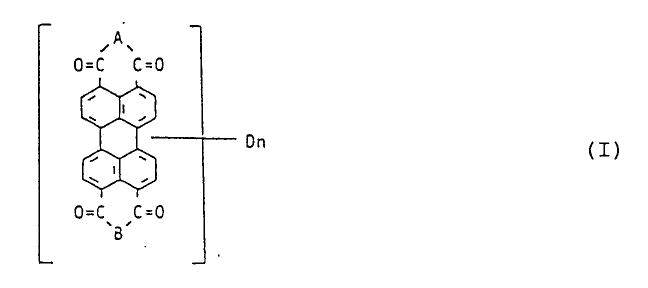 dans laquelle
A représente un radical bivalent du type 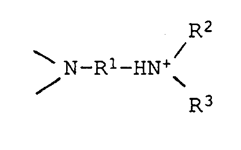 et
B représente un radical bivalent du type 〉N-R⁴-SO₃⁻ ,
n va de 0 à 8, et lorsque n > 0,
D représente un atome de chlore ou de brome, et dans la mesure où n > 1, éventuellement une combinaison de ceux-ci,
où dans les radicaux A et B ci-dessus, repésentent
R¹ un groupe alkylène avec 1 à 12 atomes de carbone, un groupe aralkylène ou un groupe arylène,
R² et R³, individuellement ainsi qu'indépendamment l'un de l'autre, chacun un atome d'hydrogène, un radical alkyle substitué ou non substitué avec 1 à 20 atomes de carbone, ou un radical alcényle substitué ou non substitué avec 2 à 20 atomes de carbone mais les deux n'étant pas simultanément l'hydrogène, ou
R² et R³, ensemble, ainsi que conjointement avec l'atome de N adjacent, forment un système hétérocyclique, qui peut éventuellement contenir encore d'autres hétéroatomes appartenant au cycle et sur lequel sont éventuellement condensés d'autres cycles, et
R⁴ représente un groupe alkylène linéaire ou ramifié avec 1 à 12 atomes de carbone.

2. Sels internes des composés de pérylène selon la revendication 1, caractérisés en ce que dans la formule générale I indiquée dans cette revendication, les symboles signifient
A et B ainsi que D et n sont caractérisés comme dans la revendication 1,
cependant au sein du principe de construction, identifié par la structure, pour les radicaux A et B,
R¹ représente un groupe alkylène en C₂-C₆, notamment le groupe éthylène ou propylène,
R² et R³, individuellement ainsi qu'indépendamment l'un de l'autre, représentent chacun un radical alkyle en C₁-C₆, notamment le radical éthyle, propyle ou butyle, qui peut éventuellement contenir encore des substituants non ioniques, ou
R² et R³, conjointement ainsi qu'ensemble avec l'atome N adjacent, forment un cycle hétérocyclique à 5 ou 6 chaînons aliphatique ou aromatique, avec chaque fois de 1 à 3 hétéroatomes identiques ou différents appartenant au cycle et un tel système de cycles contient des substituants non ioniques, ainsi que dans le cas de type aromatique, pouvant présenter aussi un cycle condensé sur le benzène, et
R⁴ représente un groupe alkylène en C₂-C₆ linéaire ou ramifié, notamment le groupe éthylène ou propylène.

3. Sels internes de composés de pérylène selon la revendication 1 ou 2, caractérisés en ce que dans la formule générale I indiquée dans cette revendication, les symboles signifient
A et B sont définis plus en détail dans les revendications 1 ou 2 et
n vaut 0.

4. Sels internes de composés de pérylène selon la revendication 1 ou 2, caractérisés en ce que dans la formule générale I indiquée dans cette revendication, les symboles signifient
A et B sont définis plus en détail comme dans les revendications 1 ou 2,
n va de 1 à 6, et
D représente un atome de chlore et/ou de brome.

5. Procédé pour la préparation des sels internes de composés de pérylène de formule générale I, définis selon l'une ou plusieurs des revendications 1 à 4, caractérisé en ce que l'on fait réagir des monoanhydrides-monoimides de l'acide pérylène-3,4,9,10-tétracarboxylique substitués sur l'azote d'imide par un élément de structure de type 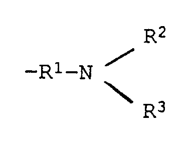 dans laquelle on attribue aux symboles R¹, R² et R³ correspondant chacun à la formule I les significations indiquées dans les revendications précédentes, avec des amines primaires aliphatiques contenant des groupes acide sulfonique de formule II :
H₂N-R⁴-SO₃H (II)
dans laquelle R⁴ a la signification identique à celle indiquée pour ce symbole conformément à la formule I dans les revendications précédentes, en solution aqueuse, dans des conditions de pH alcalin, à des températures comprises entre 50 et 200°C, de préférence entre 100 et 150°C et en ce que l'on transforme les produits de condensation obtenus de façon appropriée après avoir effectué un isolement intermédiaire par traitement avec des acides minéraux forts en les sels internes respectifs de formule I.

6. Procédé pour la préparation des sels internes de composés de pérylène de formule générale I définis selon l'une ou plusieurs des revendications 1 à 4, caractérisé en ce que l'on fait réagir des monoanhydrides-monoimides de l'acide pérylène-3,4,9,10-tétracarboxylique substitués sur l'azote d'imide par un élément de structure du type -R⁴-SO₃H dans laquelle on attribue au symbole R⁴ la signification conformément à la formule I indiquée dans les revendications précédentes, avec des alkylène-, aralkylène- ou arylènediamines de formule III présentant un groupe amino primaire : 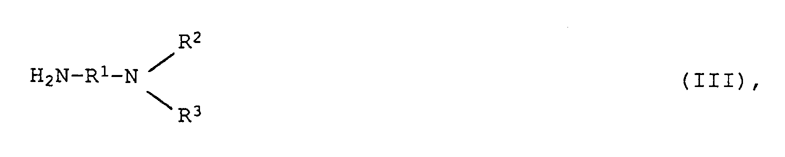 dans laquelle R¹, R² et R³ ont chacun la signification identique à celle conformément à la formule I, indiquée dans les revendications précédentes pour ces symboles, en solution aqueuse, dans des conditions de pH alcalin, à des températures comprises entre 50 et 200°C, de préférence de 80 à 120°C, et en ce que l'on transforme les produits de condensation obtenus, de façon appropriée, après avoir effectué un isolement intermédiaire par traitement avec des acides minéraux forts en les sels internes respectifs de formule I.

7. Procédé pour la préparation des sels internes de composés de pérylène de formule générale I définis selon l'une ou plusieurs des revendications 1 à 4, caractérisé en ce que l'on fait réagir des monoanhydrides-monoimides de l'acide pérylène-3,4,9,10-tétracarboxylique substitués sur l'azote d'imide par un élément de structure du type -R⁴-SO₃H dans laquelle on attribue au symbole R⁴ la signification conformément à la formule I indiquée dans les revendications précédentes, avec des alkylène-, aralkylène- ou arylènediamines de formule III présentant un groupe amino primaire : 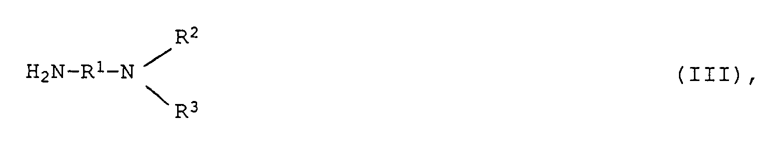 dans laquelle R¹, R² et R³ ont la signification identique à celle conformément à la formule I, indiquée pour ces symboles dans les revendications précédentes, en milieu de solvants organiques, à des températures comprises entre 150 et 230°C, de préférence de 180 à 210°C, éventuellement en présence de catalyseurs, et en ce que l'on transforme les produits de condensation obtenus de façon appropriée, après avoir effectué un isolement intermédiaire, par traitement avec des acides minéraux forts en les sels internes respectifs de formule I.

8. Utilisation des sels internes de composés de pérylène de formule générale I définis selon une ou plusieurs des revendications 1 à 4, comme colorants pour la pigmentation de matières organiques de haut poids moléculaire naturelles ou synthétiques sous forme de masses plastiques, de fontes, de solutions de filage, de vernis, d'enduits ou de pâtes d'impression.

9. Utilisation des sels internes de composés de pérylène de formule générale I, définis selon une ou plusieurs des revendications 1 à 4, comme agents de dispersion de pigments pour préparer des préparations pigmentaires.

10. Utilisation des préparations pigmentaires selon la revendication 9, dont le pigment de base dérive de la classe des composés de pérylène étant à la base de l'agent dispersant utilisé ou d'un des dérivés de cet agent.

11. Utilisation des préparations pigmentaires définies selon une ou les deux revendications 9 et 10, comme colorants pour la pigmentation de matières organiques de haut poids moléculaire naturelles ou synthétiques sous forme de masses plastiques, de fontes, de solutions de filage, de vernis, d'enduits ou de pâtes d'impression.
